# EUROPEAN PATENT APPLICATION

(11) **EP 4 400 080 A1**
(43) Date of publication of application: **17.07.2024**
(21) Application number: 24151948.7
(22) Date of filing: 15.01.2024
(51) Int. Cl.: A61F 2/36, A61F 2/30, A61F 2/46

(54) **FEMORAL TRIAL HEAD**

(30) Priority: 16.01.2023 US 202363439233 P
(71) Applicant: Zimmer, Inc., Warsaw, IN 46580 (US)
(72) Inventor: LEONARD, Tory, Warsaw, 46580 (US)
(74) Representative: Taor, Simon Edward William

(57) **Abstract**

A trial head (200) may include a proximal side (202), a distal side (204), a plurality of tabs (216) positioned at the distal side and a recess (220). The plurality of tabs collectively form an opening (218). The recess can communicate with the opening. The recess can be formed by at least a first wall portion (226) having a taper and a second wall portion (228) having a geometry that differs from the first wall portion. The second wall portion can be proximal to the first wall portion.

## Description

### CLAIM OF PRIORITY

This application claims the benefit of U.S. Provisional Patent Application Serial No. 63/439,233, filed on January 16, 2023, the benefit of priority of which is claimed hereby, and which is incorporated by reference herein in its entirety.

### FIELD

The present application is related to surgical hip replacement systems, and particularly, to femoral trial heads for use with such systems.

### BACKGROUND

Orthopedic prosthetic implants are commonly used to replace some or all of a patient's hip joint in order to restore the use of the hip joint, or to increase the use of the hip joint, following deterioration due to aging, illness, or injury due to trauma. In a total hip replacement (THR), also called a total hip arthroplasty (THA) procedure, a femoral prosthesis is used to replace a portion of the patient's femur, including the femoral neck and head. The femoral prosthesis is commonly called a stem. A portion of the femoral prosthesis is configured to be positioned within the prepared femoral canal of the patient's femur and secured via bone cement, or by a press-fit followed by bony ingrowth of the surrounding tissue into a porous coating of the stem portion. The stem can have a neck that is configured to receive a prosthetic femoral head. The femoral head is received within a prosthetic acetabular component, such as an acetabular cup received within the prepared recess of the patient's acetabulum.

### OVERVIEW

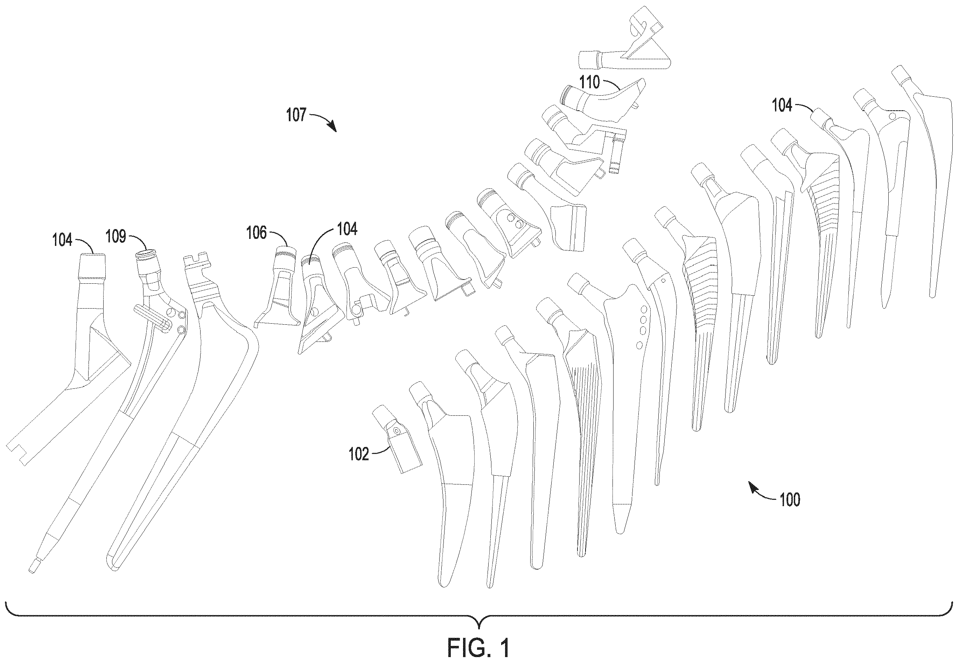

Various prosthesis systems including femoral hip prostheses each having different geometries and constructions are known. Surgeons may have a particular preference for a particular prosthesis system due to the patient's anatomy, surgical approach preference, or familiarity with the product and the surgical technique employed with the particular system. Femoral prostheses for THR may be single piece stem components or may be modular hip joint components. With modular hip joint components, the hip stem and the neck are formed as separate components. The various systems have led to a proliferation of variants. For example, the present applicant supports over 40 implant systems for hip replacement. Examples of femoral prostheses 100 of some of the systems including single piece components and necks 102 of modular systems are illustrated in FIG. 1. Each of these systems can have multiple variants for each of the femoral prostheses 100 due to different sizes, neck lengths, neck offsets, distal geometry etc.

With modular hip joint components, prior to an operation, a surgeon chooses a hip stem or a neck 102 (see FIG. 1) based on patient anatomy, body image scans, and/or other patient-specific data. During surgery, the surgeon will typically confirm this selection with trial femoral components 107 or may discover that a different hip stem or a different neck is desired to provide more optimum results. The surgeon may be provided with a number of different neck geometries (e.g., stem variants with different neck lengths, angles and/or anteversions) to accommodate various patient anatomies. These trial femoral components 107 may be single piece trial stems 109 or can be modular, typically with a trial neck component 110 that is assembled to a distal femoral trial component intraoperatively. Similarly, some trial femoral components can include a C-ring 106 (see FIG. 1) at the taper 104. However, the size (e.g. diameter) and location (e.g., depth) of the C-ring 106 can vary from system to system.

The hip prostheses are assembled to a permanent femoral head implant by a taper feature. Femoral head implants are available in various sizes, offsets, and materials. While many of the over 40 prosthesis systems fabricated, marketed and/or sold by the applicant include a 12/14 taper 104 (see FIG. 1) for coupling with a femoral head component (not shown), the geometry of such taper 104 can vary from system to system and even from stem to stem within a system. As an example, a length of the taper can differ system to system and even from stem to stem within a system.

The present inventor has recognized a trial head that can address these various geometrical differences for the implant and trial tapers 104 to eliminate the need for different head trials for the different prosthesis systems. Thus, the number of head trials that must be distributed can be reduced and cost can be saved. Additionally, the trial head described in the application can facilitate expedient and effective surgical implantation. It should be noted that the trial head discussed herein may otherwise be known as an instrument and is not implanted permanently within a patient's anatomy. Rather, the trial head is temporarily attached to the taper 104 of the femoral prosthesis 100 or the trial femoral component 107 and is placed in the joint to simulate a permanent femoral head component to check for appropriate joint kinematics such as range of motion, etc.

The present inventor also contemplates multiple systems so as to be compatible with many or all of the femoral prostheses 100 of FIG. 1 and their respective trial femoral components 107. While these head trials can have differing exterior configurations so as to simulate different sizes for the permanent femoral head component, different offsets for the permanent femoral head component, etc., all the trial heads for all the systems can include substantially a same configuration for coupling with the taper 104 of the femoral prosthesis 100 or trial femoral component 107.

### BRIEF DESCRIPTION OF THE DRAWINGS

FIG. 1 is a plan view of femoral prostheses and trial femoral components from a plurality of different prosthesis systems with which the present trial head can be used.
FIG. 2A is a plan view of a trial head according to an example of the present application.
FIG. 2B is a cross-sectional view of the trial head of FIG. 2A.
FIG. 2C is an enlarged view of the cross-section of FIG. 2B.
FIG. 3 is a cross-sectional view of part of the trial head with a recess and one of a plurality of tabs receiving a plurality of different tapers of a plurality of different trial neck components of FIG. 1.
FIG. 4 is cross-sectional view of the trial head with the recess and the plurality of tabs receiving two different tapers of two trial neck components and two different tapers of two implant neck components.
FIG. 4A is an enlarged view of a first distal part of the recess and one of the plurality of tabs of FIG. 4.
FIG. 4B is an enlarged view of a second, third, and fourth proximal part of the recess of FIG. 4.
FIGS. 5A-5C show a plurality of trial heads each having a different exterior geometry or offset but sharing substantially a same geometry for the recess and/or the plurality of tabs according to an example of the present application.
FIG. 6 shows a distal side of some of the trial heads of the systems of FIGS. 5A-5C and illustrates the same geometry for the recess and/or the plurality of tabs for some of the different configurations of the trial heads according to an example of the present application.

Corresponding reference characters indicate corresponding parts throughout the several views. The exemplifications set out herein illustrate embodiments of the invention and such exemplifications are not to be construed as limiting the scope of the invention in any manner.

### DETAILED DESCRIPTION

The present application discloses a trial head having a coupling geometry that can be utilized with any of the femoral prostheses 100 or trial femoral components 107 of FIG. 1 by being able to receive and couple with the taper 104. The trial head can form at least one interference fit with the taper 104 via a plurality of tabs, which can deflect outwards when the taper 104 is received in a recess of the trial head. Additionally, the trial head can accommodate and form a second interference fit with the C-ring 106 of the taper 104 if a C-ring is utilized.

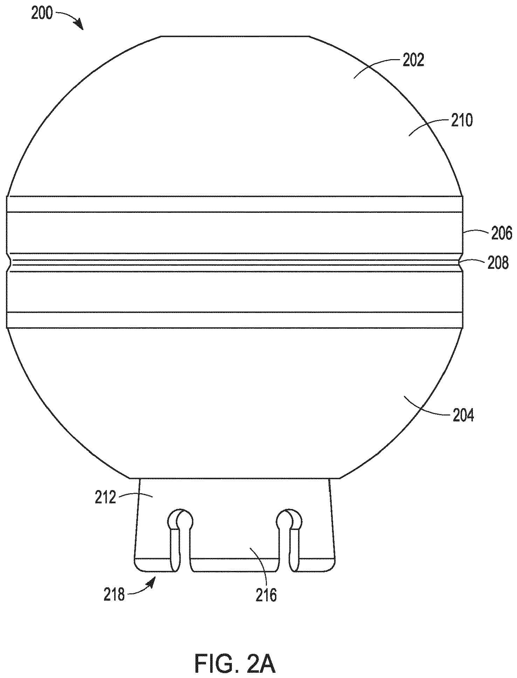

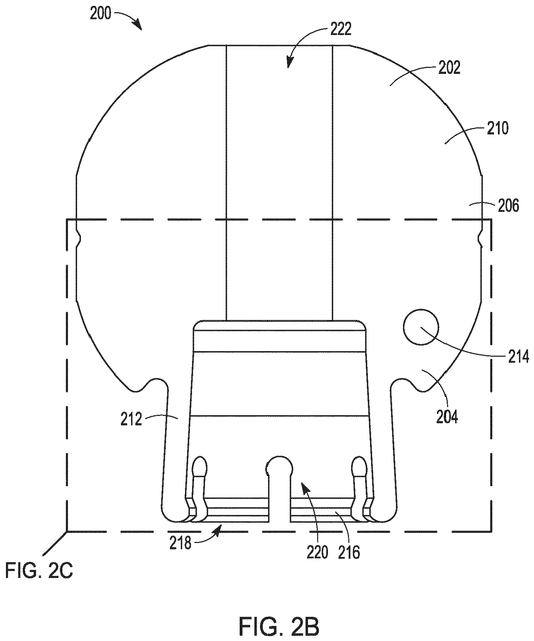

FIGS. 2A and 2B show a femoral trial head 200 according to one example. The femoral trial head 200 includes a proximal side 202 and a distal side 204. An intermediate section 206 can be positioned between the proximal side 202 and the distal side 204.

As shown in FIG. 2A, the intermediate section 206 can include a groove 208 in an exterior surface 210. The exterior surface 210 at the intermediate section 206 can be substantially flat along portions thereof. In contrast, the exterior surface 210 at the proximal side 202 and the distal side 204 can be curved so as to have a hemispherical or truncated hemispherical shape.

The groove 208 along the intermediate section 206 can be visible during imaging and can be used as a reference or indicia when imaged upon insertion of the trial head 200 into the hip joint. The groove 208 can be used to indicate if the trial head 200 is properly positioned, sized and/or can be utilized for other purposes. The imaging may be a radiographic image such as an X-ray image or fluoroscopic image, for example, or, alternatively, a computed tomography (CT) image, a magnetic resonance image (MRI), or any other suitable image.

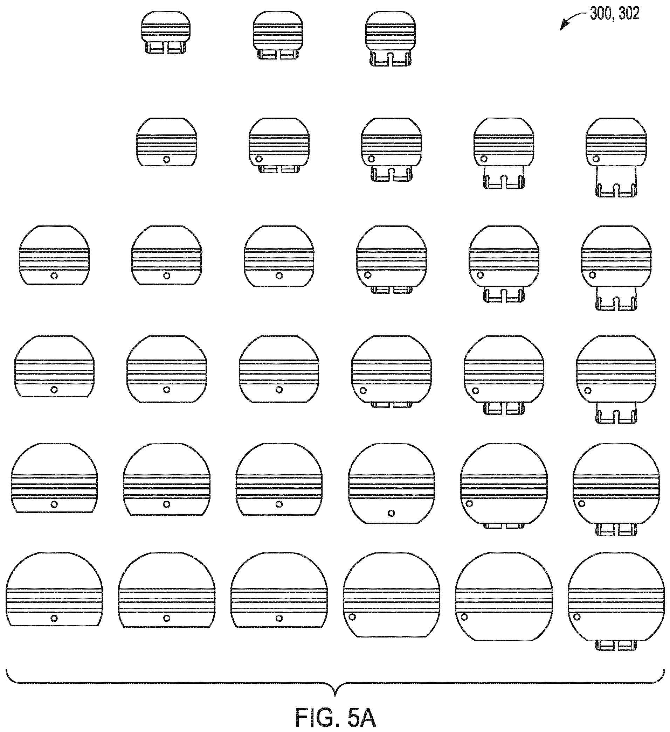

The trial head 200 of FIGS. 2A and 2B can include a skirt 212 at the distal side 204. However, the skirt 212 is not utilized in all examples of the trial head 200 (see FIGS. 5A-6 for examples that do not include the skirt 212). FIG. 2B illustrates an aperture 214 configured to receive suture.

The trial head 200 or portions thereof can be formed of a polymeric material, for example. The polymeric material can be silicone, polyphenylsulfone (PPSU), polyetheretherketone (PEEK) or other suitable biocompatible polymeric material, for example. However, other materials including composites are also contemplated for the trial head 200 or portions thereof according to further examples.

The distal side 204 can include a plurality of tabs 216. The plurality of tabs 216 can extend distally from the skirt 212 if the skirt 212 is utilized or can be recessed within the distal side 204. The plurality of tabs 216 can collectively form an opening 218 to an interior of the trial head 200. The opening 218 can be a distal-most feature of the trial head 200 and can have a distal facing orientation when coupled to the taper 104 of the femoral implant 100 or trial femoral component 107 and utilized during surgery, for example.

FIG. 2B additionally illustrates a recess 220 communicating with the opening 218. The recess 220 can extend proximally from the opening 218 internally within the trial head 200. The recess 220 can be at least partially formed by the plurality of tabs 216 at a distal region thereof. The recess 220 can communicate with an aperture 222 that extends through a remainder of the trial head 200 including to the proximal side 202 at the exterior surface 210. Together the recess 220 and the plurality of tabs 216 can be configured to couple the trial head 200 to the taper 104 of the femoral prosthesis 100 or trial femoral component 107 (see FIGS. 1) as further illustrated and described herein.

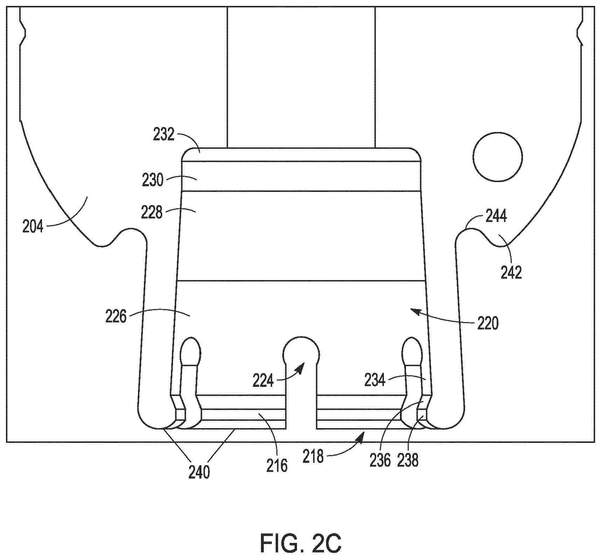

FIG. 2C shows an enlarged cross-sectional view of part of the trial head 200. As shown in FIG. 2C, each the plurality of tabs 216 are separated from adjacent of the plurality of tabs 216 by slots 224. Each of the slots 224 can have a longitudinal length in a proximal-distal direction of between 3 mm and 7 mm, for example. Each slot 224 can have a width in a circumferential direction of between 1.25 mm and 2.25 mm, for example. Each of the plurality of tabs 216 can have thickness of between 1.25 mm and 2.25 mm and a width as measured in a circumferential direction along an outside surface of between 5.5 mm and 9.5 mm and a width as measured in a circumferential direction along an inside direction of between 3.25 mm and 7.25 mm. The opening 218 can have a diameter of between 12.50 mm and 14.50 mm. The recess 220 can have a depth in a proximal-distal direction of between 12.25 mm and 15.25 mm. Due to tapering and geometry of the recess 220, a proximal end of the recess 220 can be 0.25 mm to 1.00 mm smaller in diameter than at the opening 218 due to the shape of the recess 220 (see discussion below). According to one example, there can be six of the plurality of tabs 216 arranged in a substantially equal spacing from a center of the opening 218. However, other examples of the present application contemplate the number of tabs and the other geometry discussed above and below can be modified as desired.

The recess 220 can be defined by two or more wall portions including a first wall portion 226, a second wall portion 228, a third wall portion 230 and a fourth wall portion 232. The first wall portion 226 can form a distal-most part of the recess 220. Thus, the first wall portion 226 can be at least partially formed by the plurality of tabs 216. The first wall portion 226 can have a depth of between 7.5 mm and 9.5 mm in the proximal-distal direction, for example. The first wall portion 226 can be tapered in a manner to correspond with the 12/14 taper. Thus, a cross-sectional area of the recess 220 can be reduced traveling from a distal end of the first wall portion 226 to a proximal end of the first wall portion 226.

The second wall portion 228 can have a geometry that differs from the first wall portion 226. The second wall portion 228 can be positioned proximal of the first wall portion 226 and can be connected thereto. The second wall portion 228 can include a radius of curvature rather than simply being a bore, a taper or chamfer, for example. The radius of curvature can be slight, e.g., 80 mm, for example. However, according to further examples the second wall portion 228 can be chamfered rather than having the radius of curvature. The angle of the chamfer can differ from the angle of the taper of the first wall portion 226. The radius of curvature or chamfer can result in the recess 220 reducing in cross-sectional area traveling from the distal end of the second wall portion 228 to the proximal end of the second wall portion.

The third wall portion 230 can be proximal to the second wall portion 228 and can be connected thereto. The third wall portion 230 can be cylindrically shaped (e.g., be an aperture with no radius of curvature or taper). The third wall portion 230 can be 1 mm to 2 mm in proximal-distal extent. The fourth wall portion 232 can include a radius for machining to form the recess 220 and can include the proximal end wall of the recess 220.

As shown in FIG. 2C, the plurality of tabs 216 can each have an inner wall 234 that forms the distal parts of the recess 220. The inner wall 234 can include parts of the first wall portion 226 (discussed previously), a first chamfer 236, a second chamfer 238 and a third chamfer or radius of curvature 240.

The first chamfer 236 can extend distally and inwardly from the first wall portion 226 of the recess 220 to form a restriction (a reduced diameter region of the recess 220) as compared with a distal end of the first wall portion 226. The length of the first chamfer 236 can be between 0.60 mm and 1.00 mm.

The second chamfer 238 can be connected to the first chamfer 236 and can extend distally from the first chamfer 236. The length of the second chamfer 238 can be between 0.50 mm and 0.75 mm. In some examples, the second chamfer 238 may not be chamfered in the manner shown but can be substantially flat (e.g., can be cylindrically shaped) or can be chamfered in an different direction from the direction illustrated.

The third chamfer or radius of curvature 240 can connect to the second chamfer 238 and can extend distally from the second chamfer 238 to a distal most end of the inner wall 234 (corresponding to an end of the recess 220). Indeed, the third chamfer or radius of curvature 240 can extend past the opening 218 so as to provide each of the plurality of tabs 216 with a blunt or angled distal tip and can provide a ramp for entry of the taper 104 into the recess 220. The longitudinal proximal-distal extent of the third chamfer or radius of curvature 240 can be between 0.25 mm and 0.75 mm, for example.

FIG. 2C additionally illustrates the distal side 204 adjacent the plurality of tabs 216 can include a lip 242 or other projection, and in some cases, a groove 244 to facilitate grasping of the trial head 200 for removal of the trial head 200 from the taper.

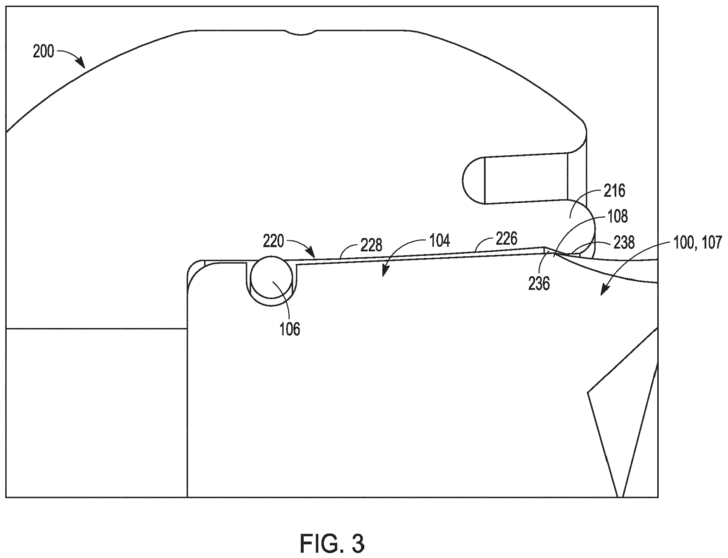

FIG. 3 shows a cross-section of a portion of the trial head 200 including part of the recess 220 and one of the plurality of tabs 216. FIG. 3 additionally illustrates the taper 104 of several of the trial femoral components 107 previously shown in FIG. 1 received by the recess 220 and engaged by the one of the plurality of tabs 216. FIG. 3 shows the C-ring 106 forming an interference fit with the second wall portion 228 that forms part of the recess 220. FIG. 3 additionally shows the one of the plurality of tabs 216 in a relaxed (neutral) state after having been flexed to accommodate assembly. Each of the plurality of tabs 216 is configured to form an interference fit with a base 108 and/or other portions of the taper 104. This interference fit between the taper 104 and the one of the plurality of tabs 216 can be at the first chamfer 236 and/or the second chamfer 238, for example.

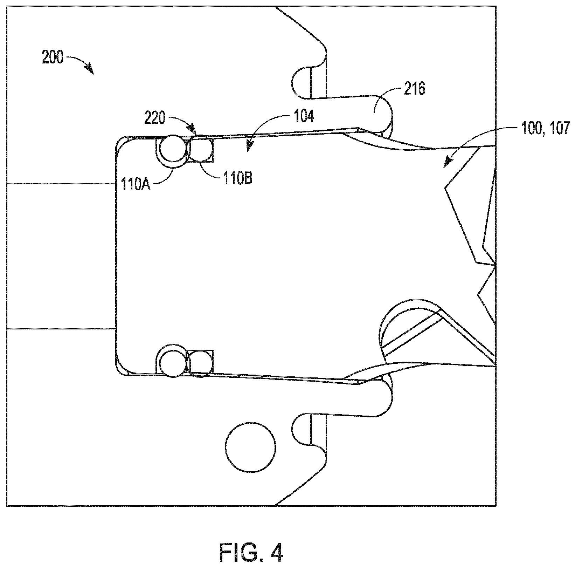

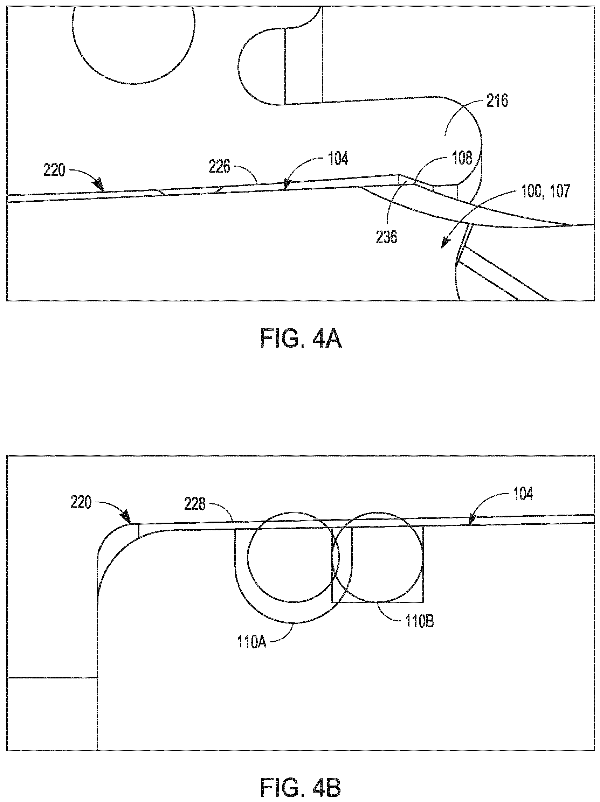

FIG. 4 shows a cross-section of a majority of the trial head 200 with four of the tapers 104 of two of the femoral prostheses 100 and two of the trial femoral components 107 of FIG. 1 received by the recess 220 and engaged by the plurality of tabs 216. Two of the four tapers shown have grooves 110A and 110B for C-rings 106. These grooves 110A and 110B are located at different relative locations and have different shapes. Thus, the C-rings 106 are located in different positions and can have different relative shapes.

FIG. 4A is an enlarged view of a distal portion of part of the recess 220 shown in FIG. 4 showing one of the plurality of tabs 216, the first wall portion 226 and first chamfer 236. The plurality of tabs 216 can be flexed outward while receiving the tapers 104 of the two femoral prostheses 100 or two femoral trial components 107. As shown in FIG. 4A, the first chamfer 236 can extend distal of the distal most base 108 of the taper 104. For this one femoral prosthesis 100, the one of the plurality of tabs 216 (and indeed all) can form an interference fit with the taper 104 at the first chamfer 236. However, FIG. 4A illustrates one additional femoral prosthesis 100 and two trial femoral components 107, for which the interference fit between the plurality of tabs 216 and the tapers 104 occurs during assembly and disassembly. This interference fit advantageously allows the trial head to retain the taper 104 of the implant stem 100 or trial component 107 to prevent inadvertent disassembly throughout the surgical procedure. The taper 104 may have a clearance fit with the first wall portion 226 and the second wall portion 228 of the recess 220. This clearance advantageously prevents a taper interlock fit and allows for unchallenging disassembly.

FIG. 4B is an enlarged view of a proximal portion of the recess 220 shown in FIG. 4 showing the second wall portion 228 arranged to correspond with the grooves 110A and 110B of the tapers 104. With the C-rings 106 received in the grooves 110A and 110B, the C-rings 106 each form an interference fit with the second wall portion 228 that forms part of the recess 220.

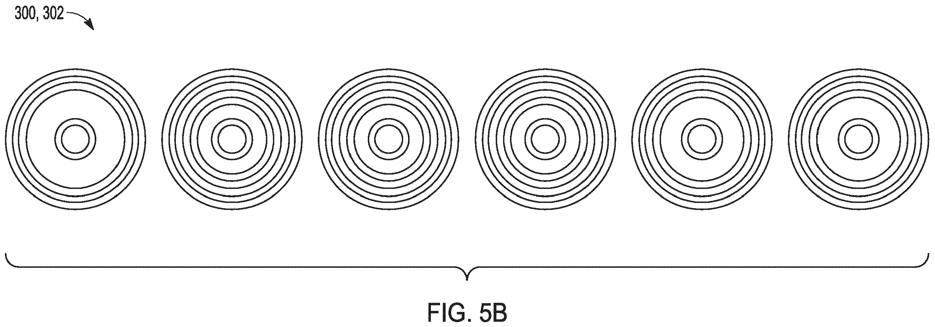

FIGS. 5A-5C show a plurality of trial heads 300 including the trial head 200 discussed previously. These plurality of trial heads 300 can be used with all of the femoral prostheses 100 and/or the trial femoral components 107 shown in FIG. 1, for example. The plurality of trial heads 300 can each include a different configuration along an exterior thereof. This different configuration can be the result of different sizes of the trial head (see FIG. 5B), different offset for the trial head, use of the skirt and/or other differences.

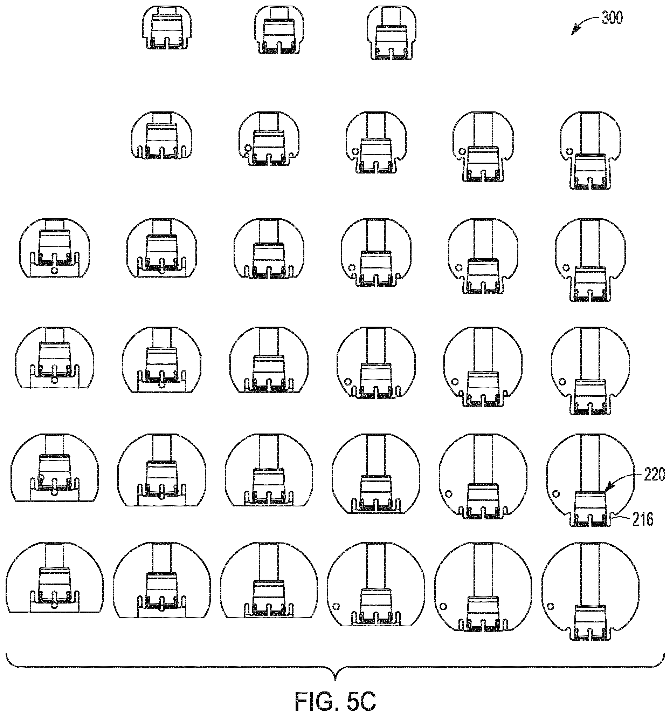

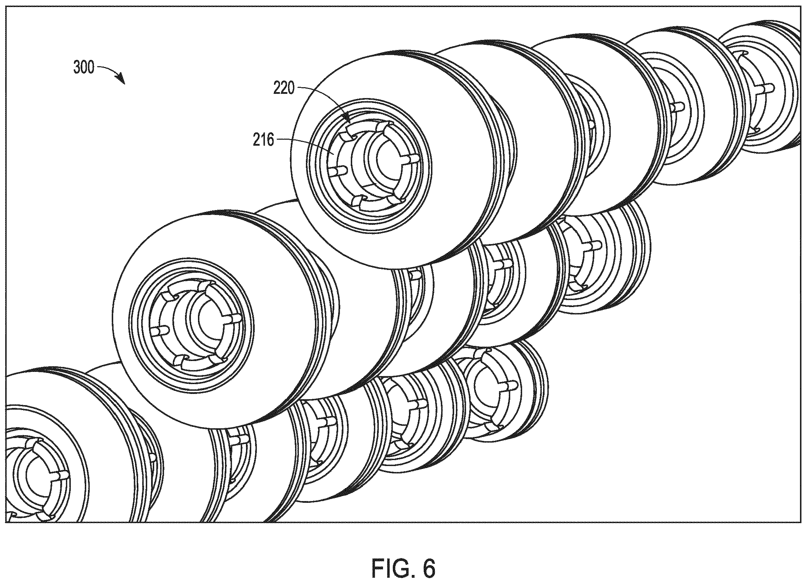

FIGS. 5C and 6 show that each of the plurality of trial heads 300 can all include substantially the same geometry for the recess 220 and the plurality of tabs 216. Thus, for example, each of the plurality of trial heads 300 the recess 220 can be of substantially the same shape including a same diameter and can have the two or more wall portions including the first wall portion 226 and the second wall portion 228 with the geometry as discussed in reference to FIG. 2C. Similarly, the inner wall 234 of the plurality of tabs 216 can have a same geometry and can include the first chamfer 236, the second chamfer 238 and/or the radius of curvature 240 as previously discussed in reference to FIG. 2C.

During the surgical operation, a trial femoral component is assembled to the trial head in order to evaluate the range of motion, leg length, soft tissue balance, etc. Alternate trial constructs may be evaluated until satisfactory performance is achieved. The corresponding femoral implant is then implanted by a conventional surgical technique. The trial head may then be assembled to the femoral prosthesis in order to perform a secondary evaluation of range of motion, leg length, soft tissue balance, etc.

The surgeon can trial the trial femoral component by coupling the taper of the stem 100 or trial femoral component 107 with the trial head as illustrated previously (see FIGS. 3-4B). Trialing is used to verify or confirm the preoperative plan and associated results. At this point, the surgeon will assess several variables, for example, leg length, offset, soft tissue balance, and anteversion, associated with the hip implant and the physical anatomy of the patient. This assessment may be completed via a conventional surgical technique, for example, moving the joint through a range of motion. The surgeon may image the trial head if desired within the hip joint using the groove as discussed previously. The surgeon may observe that more leg length is necessary, or that that the offset, soft tissue balance, and/or anteversion are unsatisfactory. The present method advantageously allows the surgeon to select a new modular neck or new stem, and, if appropriate, correspondingly change which of the plurality of trial heads 300 (see FIGS. 5A-6) is utilized. However, the coupling geometry of each of the plurality of trial heads 300 remains substantially the same reducing part number and surgical complexity. Once the surgeon has selected and implanted a specific femoral implant, the trial head may then be assembled to the femoral prosthesis in order to perform a secondary evaluation of range of motion, leg length, soft tissue balance, etc. Once trialing is completed the selected trial head can be removed and a correspondingly shaped permanent head component can be coupled to the neck via the taper.

Thus, the present application contemplates a method of trialing for a hip arthroplasty. The method can include providing a trial femoral component construct with a neck having a taper, preparing a femur to receive the femoral prosthesis, inserting the trial femoral component in the femur, coupling a first of a plurality of trial heads of different configurations to the trial femoral component by receiving the taper within a recess and capturing the taper with a plurality of tabs of the first of the plurality of trial heads, wherein the plurality of tabs are configured to flex to form an interference fit with the taper and performing a trial reduction with the first of the plurality of trial heads coupled to the trial femoral component. Optionally, the method can include implanting a femoral prosthesis with a neck having a taper simulated by the trial femoral component into the prepared femur (e.g., into the femoral canal). Optionally, rather than using the trial femoral component, the method can include coupling a trial head to a femoral prosthesis by receiving the taper in a manner similar to that described above.

Optionally, the method can include forming an interference fit within the recess between a C-ring coupled to the taper and a wall of the recess, wherein the wall has a chamfer or a radius of curvature. The method can include imaging the first of the plurality of trial heads coupled to the femoral prosthesis and referencing a groove along an outer surface of the first of the plurality of trial heads. The method can include removing the first of the plurality of trial heads from the femoral prosthesis by grasping a lip located adjacent the plurality of tabs. The coupling the first of the plurality of trial heads to the femoral prosthesis includes positioning an inner chamfered surface of each of the plurality of tabs to extend distal of a base of the taper.

Although described throughout with respect to a hip implant, the techniques could be utilized in another joint that utilizes trials such as, for example, but not limited to, shoulder arthroplasty procedures or another procedure that utilizes mating tapers for coupling of components.

Each of the following non-limiting examples (referred to as aspects and techniques below) may stand on its own, or may be combined in various permutations or combinations with one or more of the other examples. Furthermore, any element recited in the examples is optional and not a requirement of the apparatus, system or method.

### Examples:

In some aspects, the techniques described herein relate to a trial head for a hip arthroplasty including: a proximal side; a distal side; a plurality of tabs positioned at the distal side, wherein the plurality of tabs collectively form an opening; and a recess communicating with the opening, wherein the recess is formed by at least a first wall portion having a taper and a second wall portion having a geometry that differs from the first wall portion, and wherein the second wall portion is proximal to the first wall portion.

In some aspects, the techniques described herein relate to a trial head, the second wall portion has one of a radius of curvature or a chamfer.

In some aspects, the techniques described herein relate to a trial head, wherein the first wall portion is at least partially formed by the plurality of tabs.

In some aspects, the techniques described herein relate to a trial head, wherein the recess is further formed by a third wall portion that is proximal to the second wall portion, wherein the third wall portion is cylindrically shaped.

In some aspects, the techniques described herein relate to a trial head, further including an intermediate section between the proximal side and the distal side, wherein an exterior surface of the intermediate section has a groove therein.

In some aspects, the techniques described herein relate to a trial head, wherein each of the plurality of tabs includes an inner wall including: a first chamfer extending distally and inwardly from the first wall portion of the recess to form a restriction; a second chamfer extending distally from the first chamfer; and a chamfer or radius of curvature extending distally from the second chamfer to a distal most end.

In some aspects, the techniques described herein relate to a trial head, further including a femoral prosthesis or trial femoral component having a neck with a taper configured to be received by the plurality of tabs and the recess, wherein the first chamfer is configured to extend distal of a base of the taper.

In some aspects, the techniques described herein relate to a trial head, wherein the plurality of tabs flex outward to receive the taper and create an interference fit with at least a portion of the taper.

In some aspects, the techniques described herein relate to a trial head, wherein the trial femoral component incudes a C-ring at the taper, and wherein the second wall portion is configured to create an interference fit with the C-ring when the taper is received by the plurality of tabs and the recess.

In some aspects, the techniques described herein relate to a trial head, wherein at least the plurality of tabs are formed of a polymeric material.

In some aspects, the techniques described herein relate to a trial head, wherein the distal side adjacent the plurality of tabs includes a lip to facilitate removal of the trial head.

In some aspects, the techniques described herein relate to an orthopedic system for a hip arthroplasty including: a trial femoral component that includes a neck having a taper, wherein the trial femoral component represents a geometry of a femoral implant; a plurality of trial heads of different external configurations, wherein each of the plurality of trial heads is configured to couple with the trial femoral component, and wherein each of the plurality of trial heads includes: a proximal side; a distal side; a plurality of tabs positioned at the distal side, wherein the plurality of tabs collectively form an opening, wherein an inner chamfered surface of each of the plurality of tabs is configured to extend distal of a base of the taper when the trial femoral component is coupled with a respective one of the plurality of trial heads; and a recess communicating with the opening and configured to receive the taper upon insertion of the taper past the inner chamfered surface of each of the plurality of tabs.

In some aspects, the techniques described herein relate to an orthopedic system, wherein at least the recess and the plurality of tabs have a same configuration for all of the plurality of trial heads.

In some aspects, the techniques described herein relate to an orthopedic system, wherein the recess is formed by at least a first wall portion having a taper and a second wall portion having one of a chamfer or a radius of curvature, and wherein the second wall portion is proximal to the first wall portion.

In some aspects, the techniques described herein relate to an orthopedic system, wherein the first wall portion is at least partially formed by the plurality of tabs, and wherein the recess is further formed by a third wall portion that is proximal to the second wall portion, wherein the third wall portion is cylindrically shaped.

In some aspects, the techniques described herein relate to an orthopedic system, wherein the plurality of tabs are configured to flex outward to receive the taper and create an interference fit with at least a portion of the taper.

In some aspects, the techniques described herein relate to an orthopedic system, wherein the trial femoral component incudes a C-ring at the taper, and wherein a wall that defines a part of the recess with a radius of curvature that is configured to create an interference fit with the C-ring when the taper is received by the plurality of tabs and the recess.

In some aspects, the techniques described herein relate to an orthopedic system, wherein the distal side adjacent the plurality of tabs includes a lip.

In some aspects, the techniques described herein relate to a method of trialing for a hip arthroplasty including: providing a trial femoral component or femoral prosthesis with a neck having a taper; preparing a femur to receive the trial femoral component or femoral prosthesis; inserting the trial femoral component or femoral prosthesis in the femur; coupling a first of a plurality of trial heads with different external configurations to the trial femoral component or femoral prosthesis by receiving the taper within a recess and capturing the taper with a plurality of tabs of the first of the plurality of trial heads, wherein the plurality of tabs are configured to flex to form an interference fit with the taper; and performing a trial reduction with the first of the plurality of trial heads coupled to the trial femoral component or femoral prosthesis.

In some aspects, the techniques described herein relate to a method, further including forming an interference fit within the recess between a C-ring coupled to the taper and a wall of the recess, wherein the wall has one of a chamfer or a radius of curvature.

In some aspects, the techniques described herein relate to a method, further including removing the first of the plurality of trial heads from the trial femoral component or femoral prosthesis by grasping a lip located adjacent the plurality of tabs.

In some aspects, the techniques described herein relate to a method, wherein coupling the first of the plurality of trial heads to the trial femoral component or femoral prosthesis includes positioning an inner chamfered surface of each of the plurality of tabs to extend distal of a base of the taper.

### Additional Notes

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "generally" "substantially" "about" mean within 15 percent of the value provided (±). The terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) can be used in combination with each other. Other examples can be used, such as by one of ordinary skill in the art upon reviewing the above description. The Abstract is provided to comply with 37 C.F.R. § 1.72(b), to allow the reader to quickly ascertain the nature of the technical disclosure. It is submitted with the understanding that it will not be used to interpret or limit the scope or meaning of the claims. Also, in the above detailed description, various features can be grouped together to streamline the disclosure. This should not be interpreted as intending that an unclaimed disclosed feature is essential to any claim. Rather, inventive subject matter can lie in less than all features of a particular disclosed example. Thus, the following claims are hereby incorporated into the detailed description as examples or embodiments, with each claim standing on its own as a separate example, and it is contemplated that such examples can be combined with each other in various combinations or permutations. The scope of the invention should be determined with reference to the appended claims, along with the full scope of equivalents to which such claims are entitled.

## Claims

1. A trial head for a hip arthroplasty comprising:
a proximal side;
a distal side;
a plurality of tabs positioned at the distal side, wherein the plurality of tabs collectively form an opening; and
a recess communicating with the opening, wherein the recess is formed by at least a first wall portion having a taper and a second wall portion having a geometry that differs from the first wall portion, and wherein the second wall portion is proximal to the first wall portion.

2. The trial head of claim 1, the second wall portion has one of a radius of curvature or a chamfer.

3. The trial head of any one of claims 1-2, wherein the first wall portion is at least partially formed by the plurality of tabs.

4. The trial head of any one of claims 1-3, wherein the recess is further formed by a third wall portion that is proximal to the second wall portion, wherein the third wall portion is cylindrically shaped.

5. The trial head of any one of claims 1-4, further comprising an intermediate section between the proximal side and the distal side, wherein an exterior surface of the intermediate section has a groove therein.

6. The trial head of any one of claims 1-5, wherein each of the plurality of tabs includes an inner wall comprising:
a first chamfer extending distally and inwardly from the first wall portion of the recess to form a restriction;
a second chamfer extending distally from the first chamfer; and
a chamfer or radius of curvature extending distally from the second chamfer to a distal most end.

7. The trial head of claim 6, further comprising a femoral prosthesis or trial femoral component having a neck with a taper configured to be received by the plurality of tabs and the recess, wherein the first chamfer is configured to extend distal of a base of the taper.

8. The trial head of claim 7, wherein the plurality of tabs flex outward to receive the taper and create an interference fit with at least a portion of the taper.

9. The trial head of any one of claims 7-8, wherein the trial femoral component incudes a C-ring at the taper, and wherein the second wall portion is configured to create an interference fit with the C-ring when the taper is received by the plurality of tabs and the recess.

10. The trial head of any one of claims 1-9, wherein at least the plurality of tabs are formed of a polymeric material.

11. The trial head of any one of claims 1-10, wherein the distal side adjacent the plurality of tabs includes a lip to facilitate removal of the trial head.

12. The trial head of claim 1, wherein the trial head is part of an orthopedic system including a trial femoral component that includes a neck having a taper, wherein the trial femoral component represents a geometry of a femoral implant;
wherein the trial head is one of a plurality of trial heads of different external configurations, wherein each of the plurality of trial heads is configured to couple with the trial femoral component, and wherein each of the plurality of trial heads includes:
an inner chamfered surface of each of the plurality of tabs is configured to extend distal of a base of the taper when the trial femoral component is coupled with a respective one of the plurality of trial heads; and
a recess communicating with the opening and configured to receive the taper upon insertion of the taper past the inner chamfered surface of each of the plurality of tabs.

13. The orthopedic system of claim 12, wherein at least the recess and the plurality of tabs have a same configuration for all of the plurality of trial heads.

14. The orthopedic system of any one of claims 12-13, wherein the recess is formed by at least a first wall portion having a taper and a second wall portion having one of a chamfer or a radius of curvature, and wherein the second wall portion is proximal to the first wall portion, wherein the first wall portion is at least partially formed by the plurality of tabs, and wherein the recess is further formed by a third wall portion that is proximal to the second wall portion, wherein the third wall portion is cylindrically shaped.

15. The orthopedic system of any one of claims 12-14, wherein the trial femoral component incudes a C-ring at the taper, and wherein a wall that defines a part of the recess with a radius of curvature that is configured to create an interference fit with the C-ring when the taper is received by the plurality of tabs and the recess.
